# EUROPEAN PATENT SPECIFICATION

(11) **EP 0 582 183 B1**
(45) Date of publication and mention of the grant of the patent: **29.05.1996**
(21) Application number: 93111997.8
(22) Date of filing: 27.07.1993
(51) Int. Cl.: C07C 329/16, C07D 339/06

(54) **Process to prepare dithiocarbonates from trithiocarbonates**
Verfahren zur Herstellung von Dithiocarbonaten aus Trithiocarbonaten
Procédé pour la préparation des dithiocarbonates à partir des trithiocarbonates

(30) Priority: 03.08.1992 IT MI921907
(43) Date of publication of application: 09.02.1994
(73) Proprietor: CONSIGLIO NAZIONALE DELLE RICERCHE, I-00185 Roma (IT)
(72) Inventor: Barbero, Margherita, 10025 Pino Torinese, Torino (IT); Degani, Iacopo, 10122 Torino (IT); Fochi, Rita, 10122 Torino (IT); Fausone, Mara, 10127 Torino (IT)
(74) Representative: Vatti, Paolo, Dr. Ing.

(56) References cited:
- JOURNAL F]R PRAKTISCHE CHEMIE, vol. 26, no. 5-6, 1964, Leipzig, DE, pages 279 - 295 R. MAYER, ET AL.: 'Folgereaktionen des Äthylentrithiocarbonats (1,3- Dithiolan 2-thion) und seiner S-Methyl-carbenium salze. Ein Vergleich mit 1,3- dithiol thion-(2)'
- TETRAHEDRON, vol. 39, no. 10, 1983, Oxford, GB, pages 1729 - 1734 M.T.M. EL- WASSIMY, ET AL.: 'The reaction of t-butyl hypochlorite with thiocarbonyl compound - a convenient method for the C=S to C=O transformation'
- SYNTHESIS, no. 5, May 1980, Stuttgart, DE, pages 375 - 378 I. DEGANI, ET AL: 'Phase-transfer synthesis of symmetrical S,S-dialkyl dithiocarbonates via O-methyl S-alkyl dithiocarbonates'

## Description

The present invention concerns a process to prepare alkyl and arylalkyl dithiocarbonates, both acyclic and cyclic, starting from the corresponding trithiocarbonates.

The acyclic dithiocarbonates form an important class of compounds which are advantageously used for preparing on site thiols (Synthesis, 630, 1983) and thioethers (Synthesis, 1070, 1986; Chemistry and Industry, 671, 1986; EP-63 327), as well as for preparing sulphonyl chlorides and sulphonic acids (EP-234 239).

The cyclic dithiocarbonates are interesting for their industrial use, as they can be converted into compounds containing two sulphurate functions in a vicinal position, such as dithiols, disulphonyl chlorides, disulphonic acids and their derivatives. Furthermore, some derivatives of the cyclic dithiocarbonates have proved to possess a fungicide activity (J. Heterocycl. Chem., 21 (6), 1845, 1984; C.A. 88: 136601w, 1978), an antibacterial activity (C.A. 91: 74632h, 1979), a choleretic activity and an hepatoprotective activity (C.A. 74: 42345z, 1971; C.A. 71: 91459m, 1969).

There are known to be different processes to prepare dithiocarbonates.

An advantageous process (Synthesis, 365, 1978; ibidem 375, 1980; ibidem 149, 1981) provides for the use, as raw materials, of economic products such as carbon sulphide, alcohols and alkyl and arylalkyl halides, according to the following scheme:

### SCHEME 1

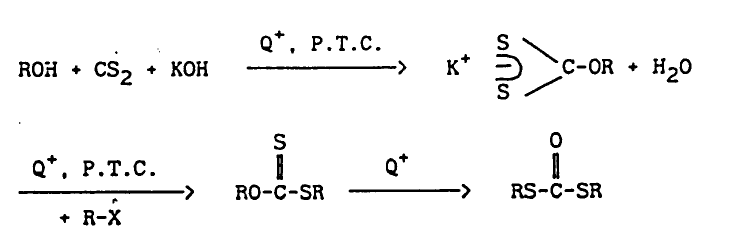

Q⁺ = onium salt
P.T.C = conditions of catalysis for phase transfer
There are also known to be processes for preparing both acyclic and cyclic dithiocarbonates wherein thiols, or dithiols, are reacted by means of reagents which are not very suited for industrial use, as being dangerous for their toxicity - such as phosgene - or being costly, such as N,N'-carbonyl-bis-imidazole (Houben-Weyl, Methoden der Organische Chemie, Georg Thieme Verlag Stuttgart Ed., N.Y., 1983, Band E4, pages 129 and 133-134).

Furthermore, such processes are evidently not convenient if wishing to use the dithiocarbonates for preparing thiols or thioethers derived therefrom.

Some processes reported in literature are based on the conversion of acyclic and cyclic trithiocarbonates into the corresponding dithiocarbonates (Houben-Weyl, Methoden der Organischen Chemie, Georg Thieme Verlag Stuttgart Ed., N.Y., 1983, Band E4, pages 133 and 139; J. Chem. Soc. 292, 1953; J. Org. Chem. 29, 360 1964; J. Chem. Soc., Perkin Trans. I 754, 1975; Chem. Ber., 113, 1898, 1980; Int. J. Sulfur chem., 8, 273, 1973; J. Am. Chem. Soc., 80, 5427, 1958; Chem. Ber., 103, 3949, 1970; C.A. 73: 77101y, 1970; C.A. 71: 112840w, 1969; C.A. 59: 9827c; C.A. 67: 99601q, 1967; C.A. 73: 77100x, 1970; C.A. 64: 19438c, 1966; Bull. Chem. Soc. Jp., 43, 168, 1970; Tetrahedron Lett., 21, 1785, 1980; J. Chem. Soc., Perkin Trans. I, 1650, 1980; Bull. chem. Soc. Jp., 59, 879, 1986).

An example of preparation, starting from trithiocarbonate, concerns an acyclic dithiocarbonate treated with t-BuOCl, so as to give the desired product in low yield and in mixture with non-identified by-products (Tetrahedron, 39, 1729, 1983).

In many cases, the processes starting from trithiocarbonates provide for the use of oxidant reagents, such as t-butyl hypochlorite, potassium permanganate, mercuric acetate, nitric acid.

One case reports the preparation of a cyclic dithiocarbonate by treating the corresponding trithiocarbonate with methyl iodide in nitromethane (both the reagent and the solvent being costly and unsuited for use on a vast scale), so as to give an intermediate carbenium S-methyl iodide which, after isolation, is converted into dithiocarbonate by hydrolysis or alcoholysis (J. Prakt. Chem. 26, 279, 1964).

The same publication also reports the treatment of the cyclic trithiocarbonate with a high excess of dimethyl sulphate: the carbenium type of intermediate thus obtained is in oily, non-crystallizable form; neither the yield obtained therewith, nor its conversion into dithiocarbonate, are reported.

The aforecited processes, making use of trithiocarbonates as raw material, are suited only for laboratory processings and, for various reasons, they have never been found of any interest for processings on a vast scale; such reasons include the lack of a suitable process for preparing trithiocarbonates, the use of reagents which are industrially prohibitive as far as costs and/or the high risks involved (as, for example, Hg(CH₃COO)₂, HgO, selenium or tellurium reagents), the modest conversion yields.

While the obstacle connected with the preparation of trithiocarbonates has been completely removed (EP-97626, in the name of the Applicant; Synthesis, 894, 1986), the same cannot be said for the difficulties connected with their conversion into dithiocarbonates.

Seen the considerable interest of dithiocarbonates for their industrial use, a constant effort is being made in seeking to obtain new preparation methods, which can be adopted on an industrial level and are economically convenient, and which allow to obtain such dithiocarbonates with a high selectivity and in high yields, starting from easy to be found and economic raw materials.

The Applicant has now surprisingly discovered that the drawbacks of known technique can be overcome with a process allowing to prepare on a vast scale, with high yields and a high selectivity, alkyl and arylalkyl dithiocarbonates, both acyclic and cyclic, starting from trithiocarbonates.

In particular, the Applicant has found that the dithiocarbonates of formula I 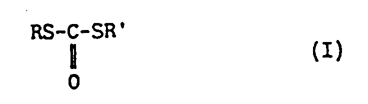
wherein,
in the case of an acyclic dithiocarbonate,
R and R' being equal, represent a linear alkyl group having 1 to 20 carbon atoms, or an arylmethyl group optionally substituted on the aromatic nucleus,
or in the case of a cyclic dithiocarbonate,
R and R' taken together, represent a -CR₁R₂CR₃R₄-, -CR₁R₂CR₃R₄CR₅R₆- group, where R₁, R₂, R₃, R₄, R₅ and R₆, being equal or different, are H, a linear or branched alkyl group having 1 to 10 carbon atoms, or an optionally substituted aromatic group, or when R and R' taken together represent a -CR₁R₂CR₃R₄- group, where R₁ and R₃ are H, then R₂ and R₄ may additionally together form a -(CH₂)₄- group,
can be obtained starting from the corresponding trithiocarbonates of formula II 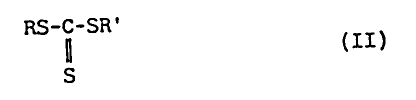
wherein R and R' are defined as above.

In the present description, by arylmethyl group is meant a group in which the aromatic part is a phenyl, a naphthyl or an aromatic heterocycle, and preferably a benzyl group.

By substituted arylmethyl group is meant a group in which the aromatic part is a phenyl, a naphthyl or an aromatic heterocycle, substituted by 1 to 3 substituents selected from the group formed by halogens and linear or branched alkyl groups having 1 to 6 carbon atoms.

By aromatic group is meant, in particular, a phenyl, a naphthyl or an aromatic heterocycle, optionally substituted by 1 to 3 groups selected from the group formed by halogens and linear or branched alkyl radicals having 1 to 6 carbon atoms.

The process according to the invention comprises the following steps:
a) The trithiocarbonate of formula (II) is treated with a compound of formula R''-X, at a temperature between 40°C and 110°C, and at the following conditions:
   - when wishing to prepare an acyclic dithiocarbonate, R''=R'=R and X is Cl, Br, -OSO₂CH₃, -OSO₂CF₃, or -OSO₂C₆H₄CH₃; R''-X is in an amount of 1 to 1.2 mols per mol of trithiocarbonate of formula (II), and the treatment is carried out in the presence of an organic acid, in an amount of 0.5 to 5 parts in volume per part in weight of said trithiocarbonate, or of an organic acid/anhydride mixture, in amounts of 0.5 to 3, and respectively, of 0.1 to 0.5 parts in volume per part in weight of said trithiocarbonate; or viceversa,
   - when wishing to prepare a cyclic dithiocarbonate, R''=CH₃ and X is -OSO₂OCH₃, or -OCOCH₃; furthermore, when X is -OSO₂OCH₃, R''-X is in an amount of 1 to 1.2 mols per mol of (II); when X is -OCOCH₃, R''-X is in an amount of 1 to 4 mols per mol of (II), and said treatment is carried out in the absence or presence of an organic acid, in an amount of preferably 0.5 to 2 parts in volume per part in weight of (II), or of an organic acid/anhydride mixture, and of an inorganic acid, in amounts of preferably 1 to 4, 0.2 to 2 and 0.5 to 4 parts in volume per part in weight, respectively, of (II).
b) The reaction mixture obtained from step a) is put under nitrogen and left to react at a temperature between 40°C and 90°C, after addition of an amount varying between 2.5 and 20 parts in volume per part in weight of trithiocarbonate (II), of a mixture of water with a mineral acid, or of an aqueous alcohol having 1 to 4 carbon atoms.

Substantially, the process according to the present invention allows the preparation of the dithiocarbonates of formula (I) starting from the diesters of the trithiocarbonic acids of formula (II), by treatment with an R''-X alkylating agent, followed by acid hydrolysis.

During step b), the RSH thiols are released: when thiol is volatile, as in the case of CH₃SH, it is carried over by a nitrogen flow into water saturated with chlorine, at a temperature of about 0°C: in this way, the thiol which has been released is converted into the corresponding alkansulphonyl chloride, as shown hereunder: 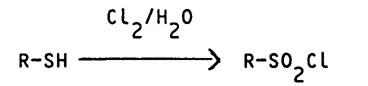
Also the non-volatile thiols can be easily separated from the raw reaction products obtained, preferably by silica gel chromatography.

The products obtained with the present process are generally recovered by extraction with organic solvents, such as diethyl ether, petroleum ether and halogenated solvents.

The raw dithiocarbonates can be obtained in practically pure form and then be used, as such, as intermediates for the intended objects; if required, the raw reaction products can be easily purified, for example by crystallization, distillation, or silica gel column chromatography.

The process according to the present invention is illustrated by the following scheme, which shows the conversions of the acyclic (II)-A and cyclic (II)-B trithiocarbonates - here represented in the form wherein R and R' are -CR₁R₂CR₃R₄- - into the respective (I)-A and (I)-B dithiocarbonates:

### SCHEME 2

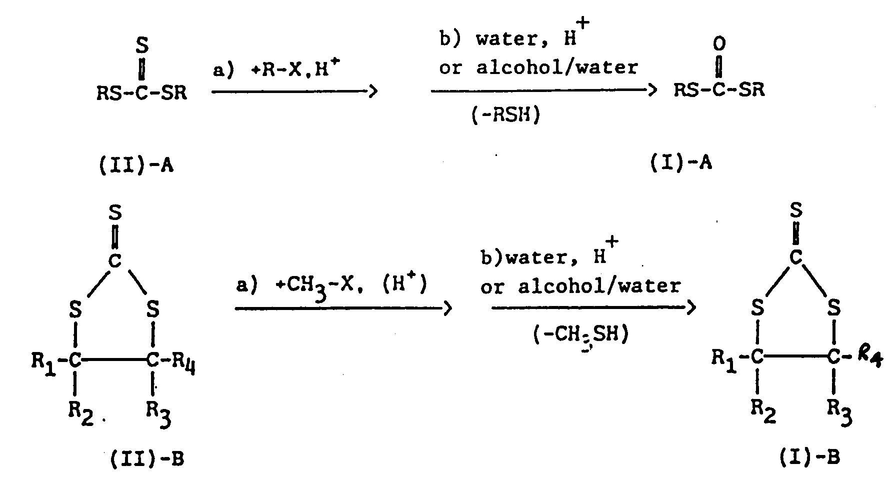

It should be noted that the treatment of the trithiocarbonates (II)-A and (II)-B with the alkylating agent, in step a), gives rise to the intermediate carbenium salts of formula (III)-A and, respectively, (III)-B. These, in step b), can be directly subjected to hydrolysis without isolation, or else they can be isolated and subsequently hydrolyzed.

(RS)₃C⁺X⁻ (III)-A

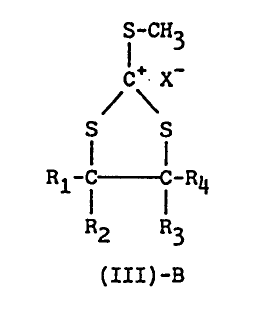

Several trithiocarbonates used as raw material in the process of the invention are known products.

Such trithiocarbonates can be prepared with the processes described in J. Am. Chem. Soc. 83, 4047, 1961.

A particularly advantageous process for their preparation on an industrial scale, and economically convenient, is described in EP-97626, in the name of the Applicant, and in Synthesis, 893, 1986 (Scheme 3):

### SCHEME 3

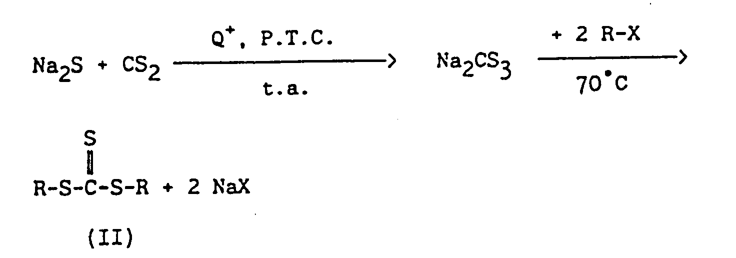

Q⁺ = onium salt
P.T.C. = conditions of catalysis for phase transfer
With said process, it is possible to prepare the trithiocarbonates (II) starting from economic raw materials, such as carbon sulphide and sodium sulphide.

The R''-X alkylating agents, preferred for the treatment of acyclic trithiocarbonates in step a), are mesylates (X=OSO₂CH₃) and arylmethyl chlorides (R''=Ar-CH₂-, X=Cl), and the organic acid used can be trifluoroacetic acid (X=OSO₂CH₃), alone or mixed with trifluoroacetic anhydride (R''=Ar-CH₂-, X=Cl).

According to a preferred embodiment of the present invention, the organic acid is trifluoroacetic acid, alone or mixed with trifluoroacetic anhydride, in a volumetric ratio varying from 10:1 to 1:1.

In step a), when wishing to prepare a cyclic dithiocarbonate using R''-X, wherein X is -OCOCH₃, the acid can be sulphuric acid, hydrochloric acid, trifluoroacetic acid, methanesulphonic acid, trifluoromethanesulphonic acid, or another strong acid and, preferably, sulphuric acid.

According to a preferred embodiment of the invention, the treatment with R''-X, wherein X is -OCOCH₃, is carried out using a mixture of sulphuric acid/acetic acid/acetic anhydride, the volume ratio of which varies from 1:1:1 to 5:5:1.

According to another preferred embodiment of the invention, when wishing to prepare a cyclic dithiocarbonate, and step a) is carried out with dimethyl sulphate (R''-X, wherein R''=CH₃ and X=OSO₂CH₃), the cyclic trithiocarbonate is mixed with the dimethyl sulphate and the mixture thus obtained is heated at the preset temperature, hence operating without solvents, diluents or acids.

According to a still preferred embodiment of the invention, said treatment with dimethyl sulphate is carried out in the presence of an amount - preferably included between 0.5 and 5 parts in volume per part in weight - of trithiocarbonate (II), of an acid selected from the group formed by sulphuric acid, hydrochloric acid, trifluoroacetic acid, methanesulphonic acid, trifluoromethanesulphonic acid, or another strong acid, preferably in the presence of trifluoroacetic acid or sulphuric acid.

According to a particularly preferred embodiment of the present invention, the treatment with dimethyl sulphate is carried out using a mixture of sulphuric acid/acetic acid/acetic anhydride, the volume ratio of which varies from 1:1:1 to 5:5:1.

Step b) is preferably carried out using water mixed with sulphuric acid, in a respective volumetric ratio varying from 9:1 to 1:1.

According to another preferred embodiment, in step b) use is made of aqueous alcohol and the alcohol is ethanol.

The process of the invention provides different advantages:
i) it has important characteristics which allow its industrial use, due to the vast scale of preparation allowed, as well as to the low cost and danger of the reagents, solvents and acids used;
ii) the two steps of the process can be carried out in succession and in a single reactor;
iii) the starting trithiocarbonates can be easily found at low costs (see Scheme 3);
iv) the conversion of the trithiocarbonates into dithiocarbonates is obtained at convenient yields, generally over 80%;
v) all the sulphur present in the trithiocarbonates can be utilized, in that the thiols which have formed as by-products can be used as such, or be converted into other useful derivatives, such as sulphonyl chlorides.

In particular, for what concerns the process reported in Scheme 1, it should be noted that said scheme is valid only for preparing acyclic dithiocarbonates, wherein the radicals are primary alkyls; it hence excludes the preparation of cyclic dithiocarbonates.

Some examples are reported hereunder to illustrate the present invention, without however limiting its scope.

The prepared dithiocarbonates have been identified by ultimate electroscopic IR and ¹-H-NMR analysis, and by comparison of their melting and boiling points to those reported in literature.

### EXAMPLE 1 - S,S-dibutyl dithiocarbonate -

a) A mixture of S,S-dibutyl trithiocarbonate (3.34 g, 0.015 mols), butyl mesylate (2.51 g, 0.0165 mols) and trifluoroacetic acid (7.5 ml), is heated to 100°C in an oil bath, under stirring. The course of the reaction is followed by ¹H-NMR analysis. The reaction is completed after about 5 hours.
b) The reaction mixture thus obtained is then treated with a flow of N₂, adding 24 ml of a solution of H₂O/H₂SO₄, in a volume ratio of 3:1, and is kept under stirring at 90°C for about 2 hours, up to complete removal of some of the intermediate products formed during the reaction (control by analysis GC-MS).

After cooling at ambient temperature, the organic phase is extracted with diethyl ether. The joined ether extracts are washed with a saturated solution of sodium bicarbonate and then with water, they are anhydrated on sodium sulphate and evaporated with a rotary evaporator. The residue, purified by column chromatography (stationary phase: silica gel; eluant: petroleum ether), gives 2.60 g of pure S,S-dibutyl dithiocarbonate (yield 85%). The product, distilled at 16 mmHg, has a boiling point of 134-135°C (Synthesis, 375, 1980: b.p. 134-135°C at 16 mmHg).

### EXAMPLE 2 - S,S-didodecyl dithiocarbonate -

a) A mixture of S,S-didodecyl trithiocarbonate (2.23 g, 0.005 mols), dodecyl mesylate (1.45 g, 0.0055 mols) and trifluoroacetic acid (5 ml), is heated to 100°C in an oil bath, under stirring. The course of the reaction is followed by ¹H-NMR analysis. The reaction is completed after about 5 hours.
b) The reaction mixture thus obtained is then treated with a flow of N₂, adding 20 ml of a solution of H₂O/H₂SO₄, in a volume ratio of 1:1, and is kept under stirring at 90°C for about 2 hours. One proceeds as described in Example 1, up to obtaining a raw product which is purified by column chromatography (stationary phase: silica gel; eluant: petroleum ether) and gives 1.74 g of pure S,S-diodecyl dithiocarbonate (yield 81%; second eluate product) and 0.84 g of dodecyl mercaptan (yield 83%; first eluate product). The S,S-didodecyl dithiocarbonate, cristallized by ethanol, has a melting point of 30-31°C (Synthesis, 375, 1980: m.p. 30+31°C).

The intermediate tris(dodecyl)methylium salt can be isolated as fluoborate and its structure can be confirmed as indicated hereinafter. To the reaction mixture obtained as described in a), there are added anhydrous acetonitrile (about 2-3 ml) and fluoboric acid with 54% ether (1 ml). To the solution, cooled in an ice and salt bath, there is added cold anhydrous diethyl ether (about 30 ml). One obtains a pale yellow precipitate formed by tris(dodecyl)methylium fluoborate, which is isolated by buckner filtration and then washed with cold anhydrous diethyl ether (3.14 g, yield 89%). The product, crystallized by anhydrous pentane/anhydrous diethyl ether, has a melting point of 40-41°C;
¹H-NMR (CDCl₃): = 0,75-1,00 ppm (m,9H,3CH₃), 1,00-1,52 (m,60H,3CH₂(CH₂)₁₀-CH₃), 2,97 (t,6H,3CH₂)-C₁₁H₂₃, J=7,00 Hz);
¹³C-NMR (CDCl₃): = 13,99 ppm (q, CH₃), 22,63, 28,69, 29,05, 29,29, 29,42, 29,58, 29,71, 30,59 (t,CH₂), 31,88 (t,CH₂-S);
The C+ band is not visible.

### EXAMPLE 3 - S,S-dibenzyl dithiocarbonate -

a) A mixture of S,S-dibenzyl trithiocarbonate (2.90 g, 0.01 mols), benzyl chloride (1.27 g, 0.01 mols), trifluoroacetic acid (6.75 ml) and trifluoroacetic anhydride (0.75 ml), is heated to 50°C in an oil bath, under stirring. The course of the reaction is followed by ¹H-NMR analysis. The reaction is completed after about 18 hours. After cooling in an ice bath, to the reaction mixture there is added fluoboric acid with 54% ether (1.5 ml), and then anhydrous diethyl ether (about 80 ml). One obtains a white precipitate formed by tris(benzylthio)methylium fluoborate, which is separated by buckner filtration and then washed with anhydrous diethyl ether (4.20 g, yield 90%).
b) To the raw tris(benzylthio)methylium fluoborate, isolated as described above, there are added 40 ml of a solution of H₂O/H₂SO₄, in a volume ratio of 9:1. The mixture is then treated with a flow of N₂ and kept under stirring at 60°C for 4 hours. One proceeds as described in Example 1, up to obtaining a raw product which is purified by column chromatography (stationary phase: silica gel; eluant: petroleum ether/diethyl ether 98:2), and gives 2.24 g of pure S,S-dibenzyl dithiocarbonate (yield 91%, from the intermediate fluoborate; yield 82%, from the starting trithiocarbonate). The product, crystallized by ethanol, has a melting point of 45-46°C (Synthesis, 375, 1980: m.p. 45-46°C).

The structure of the intermediate tris(benzylthio)methylium fluoborate, isolated as described in a), has been confirmed as indicated hereinafter. The product, crystallized by anhydrous acetonitrile/anhydrous diethyl ether, has a melting point of 103-104°C; ¹H-NMR (CF₃COOD): δ= 4,82 ppm (s,6H,3CH₂), 7,31 (m,15H,3C₆H₅); ¹³C-NMR (CF₃COOD): δ= 45,80 ppm (t,CH₂, J=150 Hz), 130,48, 131,27, 131,59, (d,C-phenyls J=155 Hz), 204,92 (s,C⁺).

### EXAMPLE 4 - S,S-dibenzyl dithiocarbonate -

a) One proceeds as described in Example 3, starting from a mixture of S,S-dibenzyl trithiocarbonate (2.90 g, 0.01 mols), benzyl chloride (1.27 g, 0.01 mols), trifluoroacetic acid (6.75 ml) and trifluoroacetic anhydride (0.75 ml), up to reaching step b).
b) To the raw tris(benzylthio)methylium fluoborate there are added 25 ml of 95° ethanol. The solution obtained is then treated with a flow of N₂ and kept under stirring at 60°C for 30 minutes. One proceeds as described in Example 3, up to obtaining 2.27 g of pure S,S-dibenzyl dithiocarbonate (yield 93%, from the intermediate fluoborate; yield 83% from the starting trithiocarbonate). The product, crystallized by ethanol, has a melting point of 45-46°C.

### EXAMPLE 5 - 1,3-dithiolan-2-one -

a) A mixture of 1,3-dithiolan-2-thione (2.04 g, 0.015 mols) and dimethyl sulphate (2.08 g, 0.0165 mols) is heated to 90°C in an oil bath, under stirring. The course of the reaction is followed by ¹H-NMR analysis. The reaction is completed after about 1 hour.
b) The reaction mixture thus obtained is then treated with a flow of N₂, adding 40 ml of a solution of H₂O/H₂SO₄, in a volume ratio of 9:1, and is kept under stirring at 90°C for 2 hours. The methyl mercaptan being formed is carried over by N₂ into water saturated with chlorine, at about 0°C, and then collected as methansulphonyl chloride. The reaction mixture obtained after removal of the methyl mercaptan is processed as described in Example 1, up to obtaining a raw product, which is purified by column chromatography (stationary phase: silica gel; eluant: petroleum ether/diethyl ether 7:3), and gives 1.60 g of pure 1,3-dithiolan-2-one (yield 89%). The product, crystallized by petroleum ether, has a melting point of 35-36°C (J. Organomet. Chem. 326, 381, 1987: m.p. 35°C).

The intermediate 2-methylthio-1,3-dithiolan-2-ylium salt can be isolated as fluoborate and its structure can be confirmed as indicated hereinafter. To the reaction mixture obtained as described in a) there are added anhydrous acetonitrile and fluoboric acid with 54% of ether. The 2-methylthio-1,3-dithiolan-2-ylium fluoborate is precipitated and isolated as described in Example 2 (3.27 g, yield 92%). The product, crystallized by anhydrous acetonitrile/anhydrous diethyl ether, has a melting point of 62-63°C;
¹H-NMR (CF₃COOD): δ= 3,20 ppm (s,3H,CH₃), 4,39 (s,4H,2CH₂); ¹³C-NMR (CF₃COOD): δ=25,08 ppm (q,CH₃,J=147 Hz), 46,43 (t,CH₂,J=154 Hz), 220,00 (s,C⁺).

### EXAMPLE 6 - 4-methyl-1,3-dithiolan-2-one -

a) A mixture of 4-methyl-1,3-dithiolan-2-thione (2.25 g, 0.015 mols) and dimethyl sulphate (2.08 g, 0.0165 mols) is heated to 90°C in an oil bath, under stirring. The course of the reaction is followed by ¹H-NMR analysis. The reaction is completed after about 1 hour.
b) The reaction mixture thus obtained is then treated with a flow of N₂, adding 25 ml of 95° ethanol, and is kept under stirring at 90°C for 2 hours. The reaction mixture is treated as described in Example 5, up to obtaining a raw reaction product formed by 4-methyl-1,3-dithiolan-2-one, which results practically pure after GC-MS analysis (1.93 g, yield 96%). The product, distilled at 10 mmHg, has a boiling point of 113-114°C (Obshch. Khim., 32, 2940, 1962: b.p. 82-85°C at 2 mmHg).

The intermediate 4-methyl-2-methylthio-1,3-dithiolan-2-ylium can be isolated as perchlorate and its structure can be confirmed as indicated hereinafter. To the reaction mixture obtained as described in a) there are added anhydrous acetonitrile (about 2-3 ml) and a 70% perchloric acid solution (2 ml). The mixture is cooled in an ice and salt bath, and cold anhydrous diethyl ether (about 40 ml) is then added thereto. One obtains a white precipitate formed by 4-methyl-2-methylthio-1,3-dithiolan-2-ylium perchlorate, which is isolated by buckner filtration and then washed with anhydrous diethyl ether (1.98 g, yield 75%). The product, crystallized by anhydrous acetonitrile/anhydrous diethyl ether, has a melting point of 65-66°C; ¹H-NMR (CF₃COOD): δ=1,80 ppm (d,3H,CH₃, J=7,00 Hz), 3,20 (s,3H,S-CH₃), 4,02-4,65 (m,2H,CH₂), 4,91-5,25 (m,1H,CH); ¹³C-NMR (CF₃COOD): δ= 17,96 ppm (q,CH₃, J=132 Hz), 25,50 (q,S-CH₃, J=146 Hz), 52,94 (t,CH₂, J=155 Hz), 61,16 (d,CH,J=148 Hz), 213,92 (s,C⁺).

### EXAMPLE 7 - 4-Decyl-1,3-dithiolan-2-one -

a) To a solution of methyl acetate (2.22 g, 0.03 mols) in acetic anhydride (2.80 g), cooled in an ice bath, there is added - slowly and under stirring - a solution of glacial acetic acid (7 ml) and sulphuric acid (7 ml), previously cooled. To the solution thus obtained there are subsequently added, under stirring, 2.77 g (0.01 mols) of 4-decyl-1,3-dithiolan-2-thione. The reaction mixture is slowly heated, in an oil bath, up to 80°C in about 20 minutes. Said temperature is kept for two hours. As the reaction continues, one notices that the initial yellow emulsion turns into a green colour and yellow spongy-looking clots start to separate therefrom.
b) The reaction mixture thus obtained is then treated with a flow of N₂, adding 40 ml of a solution of H₂O/H₂SO₄, in a volume ratio of 9:1, and is kept under stirring at 80°C for 1.5 hours. The reaction mixture is treated as described in Example 5, up to obtaining a raw product which is purified by column chromatography (stationary phase: silica gel; eluent: petroleum ether/acetone 98:2), giving 2.30 g of pure 4-decyl-1,3-dithiolan-2-one (yield 89%). The product, distilled at 2 mmHg, has a boiling point of 197-198°C. The structure of the compound has been confirmed by NMR and IR spectroscopy:
   ¹H-NMR (CDCl₃): δ = 0,62-0,90 ppm (m,3H, CH₃), 0,90-1,34 (m,16H,(CH₂)₄), 1,61-1,90 (m,2H,CH₂-C₉H₁₉), 3,15-3,70 (m,2H,CH₂-S), 3,70-4,15 (m,1H,CH-S); ¹³C-NMR (CDCl₃): δ = 13,94 ppm (q,CH₃), 22,53, 28,02, 29,20, 29,37, 31,75, 34,55 (t,CH₂), 41,06 (t,CH₂-S), 53,49 (d,CH-S), 194,90 (s,C=O); IR (CCl4): 1660 cm⁻¹ (C=O).

The intermediate 4-decyl-2-methylthio-1,3-dithiolan-2-ylium salt can be isolated as fluoborate and its structure can be confirmed as indicated hereinafter. To the reaction mixture, obtained as described in a), there are added anhydrous acetonitrile and fluoboric acid with 54% ether. Proceeding as described in Example 2, one isolates the 4-decyl-2-methylthio-1,3-dithiolan-2-ylium fluoborate, with a yield of 74% (2.76 g). The product, crystallized by anhydrous acetonitrile/anhydrous diethyl ether, has a melting point of 40-41°C; ¹H-NMR (CF₃COOD): δ= 0,80 ppm (m,3H,C₉H₁₈-CH₃), 1,21-1,62 (m,16H,8CH₂), 1,96-2,25 (m,2H,CH₂-C₉H₁₉), 3,20 (s,S-CH₃), 4,10-4,47 (m,2H,CH₂-S), 4,75-5,07 (m,1H,CH); ¹³C-NMR (CH₃COOD): δ= 14,51 ppm (q,CH₃), 25,36 (q,S-CH₃), 24,10, 29,88, 30,51, 30,69, 30,84, 30,93, 31,04, 33,50, 33,58 (t,CH₂), 50,89 (t,S-CH₂), 66,99 (d,CH), 214,47 (s,C⁺).

### EXAMPLE 8 - 4,5-dimethyl-1,3-dithiolan-2-one -

a) To a solution of methyl acetate (2.22 g, 0.03 mols), acetic anhydride (2.80 g), glacial acetic acid (7 ml) and sulphuric acid (7 ml) - prepared as described in Example 7 and previously cooled - there are added, under stirring, 1.64 g (0.01 mols) of 4,5-dimethyl-1,3-dithiolan-2-thione. The reaction mixture is slowly heated, in an oil bath, up to 60°C for about 20 minutes. Said temperature is maintained for 2 hours.
b) The reaction mixture thus obtained is then treated with a flow of N₂, adding 40 ml of a solution of H₂O/H₂SO₄, in a volume ratio of 9:1, and is kept under stirring at 60°C for 2 hours. One then proceeds as described in Example 1, up to obtaining a raw product which is purified by column chromatography (stationary phase: silica gel; eluent: petroleum ether/diethyl ether 90:10), and gives 1.11 g of pure 4,5-dimethyl-1,3-dithiolan-2-one (yield 75%). The product, distilled at 12 mmHg, has a boiling point of 120°C. Analyzed by GC, it forms a cis and trans mixture in a 1:3 proportion.

The two diastereoisomers have been separated by repeated column chromatographies (stationary phase: silica gel; eluent: petroleum ether/diethyl ether 90:10). The trans isomer, crystallized by petroleum ether, has a melting point of 41-42°C (J. Org. Chem. 29, 360, 1964: m.p. 41-41.5°C);
¹H-NMR (CD₃COOD₃): = 1.55 ppm (d,6H,2CH₃,J = 6.00 Hz), 3.92 (8 lines, 2H,2CH, J(H,CH₃)) = 6.12 Hz, J(H,H = 1.50); ¹³C-NMR (CDC1₃): = 13.90 ppm (q,CH₃, J = 127 Hz), 54.03 (d,CH-S J = 143 Hz), 195,87 (s,C = O); IR CCl₄): 1662 cm-1 (C = O).
The cis isomer is an oil (J. Org. Chem. 29, 360, 1964: non-solidifiable);
¹H-NMR (CD₃COCD₃): δ = 1.41 ppm (d,6H,2CH₃,J = 6.40 Hz); ¹³C-NMR (CDCl₃): δ = 15.64 (q,CH₃), 51.39 (d,CH-S).
The CO band is not visible; IR (CCl₄): 1660 cm⁻¹ (C = O).

The intermediate 4,5-dimethyl-2-methylthio-1,3-dithiolan-2-ylium salt can be isolated as fluoborate and its structure can be confirmed as indicated hereinafter. To the reaction mixture obtained as described in a) there is added anhydrous acetonitrile and then fluoboric acid with 54% ether. By proceeding as described in Example 2, one isolates the 4,5-dimethyl-2-methylthio-1,3-dithiolan-2-ylium fluoborate, obtaining a 90% yield (2.39 g). The product, crystallized by anhydrous acetonitrile/anhydrous diethyl ether, has a melting point of 63-64°C;
¹H- NMR (CF₃COOD): δ = 1,73 ppm (d,6H,2CH₃, J=7,00 Hz), 3,17 (s,3H,S-CH₃), 4,55-5,09 (m,2H,2CH); ¹³C-NMR (CF₃COOD); δ = 13,34, 18,05 ppm (q,CH₃,J=130 Hz), 25,26, 25,45 (q,S-CH₃, J=130), 64,07, 66,82 (d,CH, J=145), 214,22 (s,C⁺).

### EXAMPLE 9 - 4,5-tetramethylene-1,3-dithiolan-2-one -

a) A mixture of 4,5-tetramethylene-1,3-dithiolan-2-thione (1.90 g, 0.01 mols), dimethyl sulphate (1.39 g, 0.011 mols) and trifluoroacetic acid (2.5 ml) is heated to 90°C in an oil bath, under stirring. The course of the reaction is followed by ¹H-NMR analysis. The reaction is completed after about 3 hours.
b) The reaction mixture thus obtained is then treated with a flow of N₂, adding 25 ml of 95% ethanol, and is kept under stirring at 90°C for 1 hour. The reaction mixture is treated as described in Example 5, up to obtaining a raw product which is purified by column chromatography (stationary phase: silica gel; eluent: petroleum ether/diethyl ether 9:1), and gives 1.32 g of pure 4,5-tetramethylene-1,3-dithiolan-2-one (yield 76%). The product, crystallized by ethanol, has a melting point of 109-110°C.

The intermediate 2-methylthio-4,5-tetramethylene-1,3-dithiolan-2-ylium salt can be isolated as fluoborate and its structure can be confirmed as indicated hereinafter. To the reaction mixture obtained as described in a) there is added anhydrous acetonitrile and then fluoboric acid with 54% ether. By proceeding as described in Example 2, one isolates the 2-methylthio-4,5-tetramethylene-1,3-dithiolan-2-ylium, obtaining an 89% yield (2.60 g). The product, crystallized by anhydrous acetonitrile/anhydrous diethyl ether, has a melting point of 116-117°C;
¹H-NMR (CF₃COOD): δ = 1,25-1,75, 1,75-2,29, 2,42-2,77 ppm (3m, 1:2:1, 8H, 4CH₂), 3,19 (s,3H,CH₃), 4,19-4,47 (m,2H,2CH-S); ¹³C-NMR (CF₃COOD): δ = 24,96 ppm (q,CH₃, J=145 Hz), 25,56, 30,05 (t,CH₂,J=135), 68,95 (d,C-S, J=150), 221,10 (s,C⁺).

### EXAMPLE 10 - 4,5-tetramethylene-1,3-dithiolan-2-one -

a) A mixture of 4,5-tetramethylene-1,3-dithiolan-2-thione (1.90 g, 0.01 mols), dimethyl sulphate (1.39 g, 0.011 mols) and of an 8.5 ml solution consisting of glacial acetic acid/acetic anhydride/sulphuric acid (in volume ratios of 5/1/2.5), is heated to 80°C in an oil bath, under stirring. The course of the reaction is followed by ¹H-NMR analysis. The reaction is completed after 1.5 hours. During the reaction, one may notice an initial decolorization, with formation of spongy-looking yellow blots, and a final browning.
b) The reaction mixture thus obtained is then treated with a flow of N₂, adding 40 ml of H₂O/H₂SO₄ in a volume ratio of 9:1, and is kept under stirring at 80°C for 1.5 hours. The reaction mixture is treated as described in Example 5, up to obtaining a raw product which is purified by column chromatography (stationary phase: silica gel; eluent: petroleum ether/diethyl ether 9:1), and gives 1.50 g of pure 4,5-tetramethylene-1,3-dithiolan-2-one (yield 86%). The product, crystallized by ethanol, has a melting point of 109-110°C.

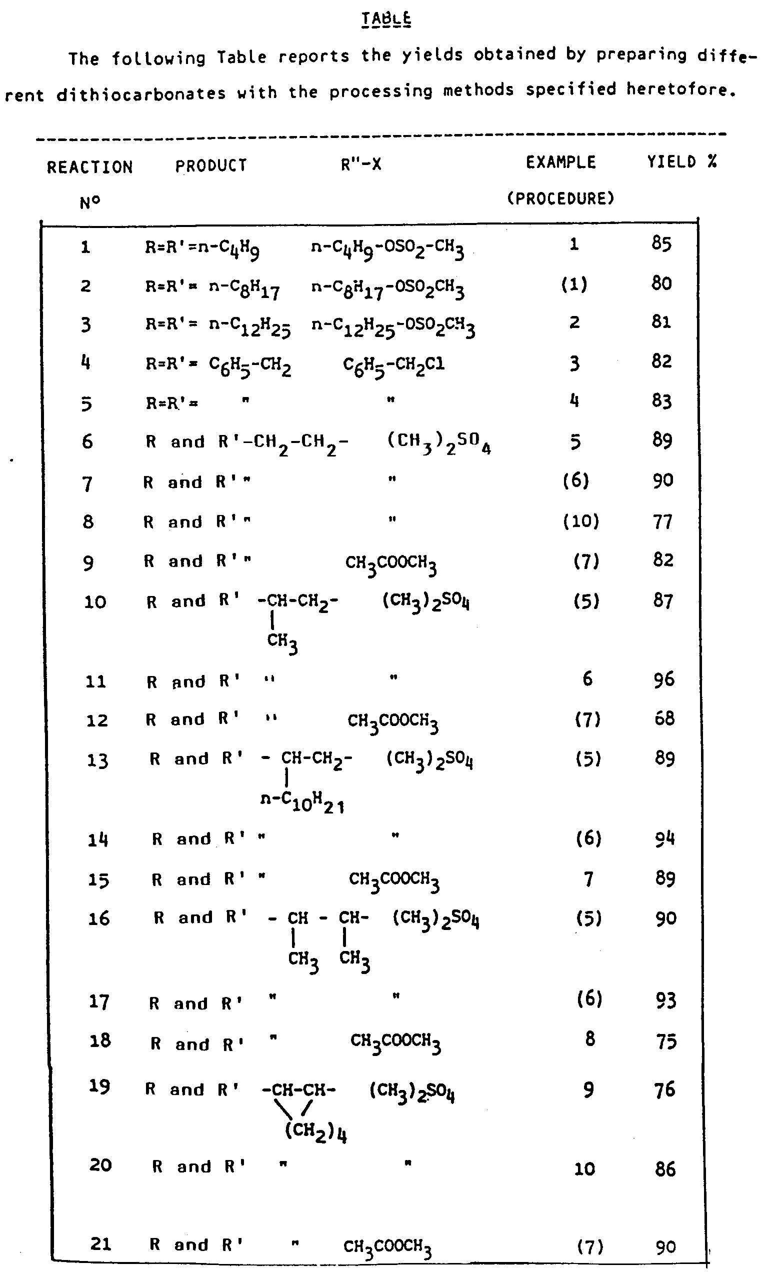

## Claims

1. Process to prepare both acyclic and cyclic dithiocarbonates of formula (I) 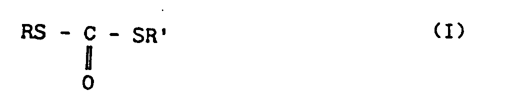 wherein,
in the case of an acyclic dithiocarbonate,
R and R' being equal, represent a linear alkyl group having 1 to 20 carbon atoms, or an arylmethyl group optionally substituted on the aromatic nucleus,
or in the case of a cyclic dithiocarbonate,
R and R' taken together, represent a -CR₁R₂CR₃R₄-, -CR₁R₂CR₃R₄CR₅R₆-group, where R₁, R₂, R₃, R₄, R₅ and R₆, being equal or different, are H, a linear or branched alkyl group having 1 to 10 carbon atoms, or an optionally substituted aromatic group, or when R and R' taken together represent a -CR₁R₂CR₃R₄- group, where R₁ and R₃ are H, then R₂ and R₄ may additionally together form a -(CH₂)₄- group,
starting from the corresponding trithiocarbonate, which comprises following steps:
a) The corresponding trithiocarbonate of formula (II) 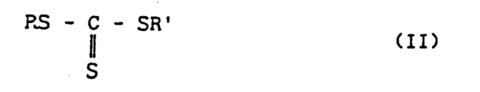 wherein R and R' are defined as above,
is treated with a compound of formula R''-X, at a temperature between 40°C and 110°C, and at the following conditions:
- when wishing to prepare an acyclic dithiocarbonate, R''=R'=R and X is Cl, Br, -OSO₂CH₃, -OSO₂CF₃, or -OSO₂C₆H₄CH₃; R''-X is in an amount of 1 to 1.2 mols per mol of trithiocarbonate of formula (II), and the treatment is carried out in the presence of an organic acid, in an amount of 0.5 to 5 parts in volume per part in weight of said trithiocarbonate, or of an organic acid/anhydride mixture, in amounts of 0.5 to 3, and respectively, of 0.1 to 0.5 parts in volume per part in weight of said trithiocarbonate, or viceversa,
- when wishing to prepare a cyclic dithiocarbonate, R''=CH₃ and X is -OSO₂OCH₃, or -OCOCH₃; furthermore, when X is -OSO₂OCH₃, R''-X is in an amount of 1 to 1.2 mols per mol of (II); when X is -OCOCH₃, R''-X is in an amount of 1 to 4 mols per mol of (II), and said treatment is carried out in the absence or presence of an organic acid, in an amount of 0.5 to 2 parts in volume per part in weight of (II), or of an organic acid/anhydride mixture, and of an inorganic acid, in amounts of 1 to 4, 0.2 to 2 and 0.5 to 4 parts in volume per part in weight, respectively, of (II).
b) The reaction mixture obtained from step a) is put under nitrogen and left to react at a temperature between 40°C and 90°C, after addition of an amount varying between 2.5 and 20 parts in volume per part in weight of trithiocarbonate (II), of a mixture of water with a mineral acid, or of an aqueous alcohol having 1 to 4 carbon atoms.

2. Process as in claim 1), characterized in that, to obtain acyclic dithiocarbonates, in step a) the acyclic trithiocarbonates are treated with R''-X compounds selected among mesylates and arylmethyl chlorides, and the organic acid used is trifluoroacetic acid, alone or mixed with trifluoroacetic anhydride.

3. Process as in claim 1), characterized in that, to obtain cyclic dithiocarbonates, in step a) the cyclic trithiocarbonate is mixed with R''-X, wherein X is -OCOCH₃, and the acid is sulphuric acid, hydrochloric acid, trifluoroacetic acid or trifluoromethanesulphonic acid.

4. Process as in claim 1), characterized in that, when wishing to prepare a cyclic dithiocarbonate and step a) is carried out using, as R''-X, dimethyl sulphate, the reaction is carried out in the absence of solvents, diluents or acids.

5. Process as in claim 1), wherein said treatment with R''-X = dimethyl sulphate is carried out in the presence of an acid selected from the group formed by sulphuric acid, hydrochloric acid, trifluoroacetic acid, methanesulphonic acid and trifluoromethanesulphonic acid.

6. Process as in claim 1), characterized in that, when wishing to prepare a cyclic dithiocarbonate and step a) is carried out using, as R''-X, dimethyl sulphate or methyl acetate, the reaction is carried out in the presence of a mixture of sulphuric acid/acetic acid/acetic anhydride in a volume ratio varying from 1:1:1 to 5:5:1.

7. Process as in claim 1), wherein step b) is carried out using water mixed with sulphuric acid in a respective volumetric ratio varying from 9:1 to 1:1.

8. Process as in claim 1) wherein, in step b), the alcohol is ethanol.

9. Process as in claim 1) wherein, when a volatile thiol is released, it is carried over by a nitrogen flow into water saturated with chlorine, at a temperature of about 0°C, so as to be converted into the corresponding alkansulphonyl chloride.

## Patentansprüche

1. Verfahren zur Herstellung von sowohl acyclischen als auch cyclischen Dithiocarbonaten der Formel (I) 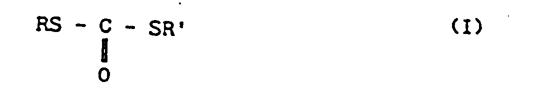 in der,
im Falle eines acyclischen Dithiocarbonates,
R und R', die gleich sind, eine lineare Alkylgruppe mit 1 bis 20 Kohlenstoffatomen oder eine Arylmethylgruppe, die fakultativ am aromatischen Kern substituiert ist, darstellen,
oder im Falle eines cyclischen Dithiocarbonates,
R und R' zusammengenommen eine -CR₁R₂CR₃R₄-, -CR₁R₂CR₃R₄CR₅R₆-Gruppe, darstellen, wobei R₁, R₂, R₃, R₄, R₅ und R₆, die gleich oder verschieden sind, H, eine lineare oder verzweigte Alkylgruppe mit 1 bis 10 Kohlenstoffatomen oder eine fakultativ substituierte aromatische Gruppe sind, oder wobei, wenn R und R' zusammengenommen eine -CR₁R₂CR₃R₄-Gruppe darstellen, wobei R₁ und R₃ H sind, dann R₂ und R₄ zusätzlich zusammen eine -(CH₂)₄-Gruppe bilden können,
ausgehend von dem entsprechenden Trithiocarbonat, welches die folgenden Schritte umfaßt:
a) Das entsprechende Trithiocarbonat der Formel (II) 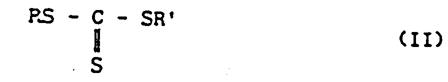 in der R und R' wie oben definiert sind,
wird mit einer Verbindung der Formel R''-X bei einer Temperatur zwischen 40°C und 110°C und bei den folgenden Bedingungen behandelt,
- wenn man ein acyclisches Dithiocarbonat herstellen möchte, ist R''=R'=R und X Cl, Br, -OSO₂CH₃, -OSO₂CF₃, oder -OSO₂C₆H₄CH₃; liegt R''-X in einer Menge von 1 bis 1,2 Mol pro Mol Trithiocarbonat der Formel (II) vor und wird die Behandlung durchgeführt in der Gegenwart einer organischen Säure, in einer Menge von 0,5 bis 5 Volumenteilen pro Gewichtsteil besagten Trithiocarbonates, oder einer Mischung von organischer Säure und Anhydrid, in Mengen von 0,5 bis 3 bzw. 0,1 bis 0,5 Volumenteilen pro Gewichtsteil besagten Trithiocarbonates,
oder umgekehrt,
- wenn man cyclisches Dithiocarbonat herstellen möchte, ist R''=CH₃ und X -OSO₂OCH₃ oder -OCOCH₃; liegt außerdem, wenn X -OSO₂OCH₃ ist, R''-X in einer Menge von 1 bis 1,2 Mol pro Mol (II) vor; liegt, wenn X -OCOCH₃ ist, R''-X in einer Menge von 1 bis 4 Mol pro Mol (II) vor und wird besagte Behandlung durchgeführt in der Abwesenheit oder Gegenwart einer organischen Säure, in einer Menge von 0,5 bis 2 Volumenteilen pro Gewichtsteil (II), oder einer Mischung aus organischer Säure und Anhydrid und einer anorganischen Säure, in Mengen von 1 bis 4, 0,2 bis 2 bzw. 0,5 bis 4 Volumenteilen pro Gewichtsteil (II).
b) Die Reaktionsmischung, die aus Schritt a) erhalten wird, wird unter Stickstoff gegeben und bei einer Temperatur zwischen 40°C und 90°C nach Zugabe einer Menge, die zwischen 2,5 und 20 Volumenteilen pro Gewichtsteil Trithiocarbonat (II) variiert, einer Mischung von Wasser mit einer Mineralsäure oder eines wäßrigen Alkohols mit 1 bis 4 Kohlenstoffatomen reagieren gelassen.

2. Verfahren nach Anspruch 1, dadurch gekennzeichnet, daß, um acyclische Dithiocarbonate zu erhalten, in Schritt a) die acyclischen Trithiocarbonate mit R''-X-Verbindungen behandelt werden, die ausgewählt sind aus Mesylaten und Arylmethylchloriden, und daß die verwendete organische Säure Trifluoressigsäure ist, allein oder vermischt mit Trifluoressigsäureanhydrid.

3. Verfahren nach Anspruch 1, dadurch gekennzeichnet, daß, um cyclische Dithiocarbonate zu erhalten, in Schritt a) das cyclische Trithiocarbonat mit R''-X vermischt wird, wobei X -OCOCH₃ ist, und die Säure Schwefelsäure, Salzsäure, Trifluoressigsäure oder Trifluormethansulfonsäure ist.

4. Verfahren nach Anspruch 1, dadurch gekennzeichnet, daß, wenn man ein cyclisches Dithiocarbonat herstellen möchte und Schritt a) unter Verwendung von Dimethylsulfat, als R''-X, durchgeführt wird, die Reaktion in der Abwesenheit von Lösungsmitteln, Verdünnungsmitteln oder Säuren durchgeführt wird.

5. Verfahren nach Anspruch 1, wobei besagte Behandlung mit R''-X = Dimethylsulfat in der Gegenwart einer Säure durchgeführt wird, die ausgewählt ist aus der Gruppe, die aus Schwefelsäure, Salzsäure, Trifluoressigsäure, Methansulfonsäure und Trifluormethansulfonsäure gebildet ist.

6. Verfahren nach Anspruch 1, dadurch gekennzeichnet, daß, wenn man ein cyclisches Dithiocarbonat herstellen möchte und Schritt a) unter Verwendung von Dimethylsulfat oder Methylacetat, als R''-X, durchgeführt wird, die Reaktion in der Gegenwart einer Mischung aus Schwefelsäure/Essigsäure/Essigsäureanhydrid in einem Volumenverhältnis, das von 1:1:1 bis 5:5:1 variiert, durchgeführt wird.

7. Verfahren nach Anspruch 1, wobei Schritt b) unter Verwendung von Wasser durchgeführt wird, das mit Schwefelsäure in einem entsprechenden volumetrischen Verhältnis vermischt ist, das von 9:1 bis 1:1 variiert.

8. Verfahren nach Anspruch 1, wobei, in Schritt b), der Alkohol Ethanol ist.

9. Verfahren nach Anspruch 1, wobei, wenn ein flüchtiges Thiol freigesetzt wird, es durch einen Stickstoffstrom in Wasser überführt wird, das mit Chlor gesättigt ist, bei einer Temperatur von etwa 0°C, um in das entsprechende Alkansulfonylchlorid umgewandelt zu werden.

## Revendications

1. Procédé de préparation de dithiocarbonates acycliques et de dithiocarbonates cycliques de formule (I) 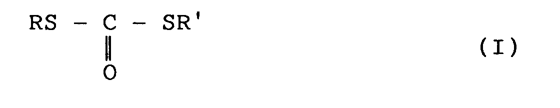 où,
dans le cas d'un dithiocarbonate acyclique,
R et R', qui sont identiques, représentent un groupe alkyle linéaire ayant de 1 à 20 atomes de carbone ou un groupe arylméthyle éventuellement substitué sur le noyau aromatique,
ou dans le cas d'un dithiocarbonate cyclique,
R et R' pris ensemble représentent un groupe -CR₁R₂CR₃R₄-ou -CR₁R₂CR₃R₄CR₅R₆-, où R₁, R₂, R₃, R₄, R₅ et R₆, qui peuvent être identiques ou différents, représentent l'hydrogène, un groupe alkyle linéaire ou ramifié ayant de 1 à 10 atomes de carbone ou un groupe aromatique éventuellement substitué, ou quand R et R' pris ensemble représentent un groupe -CR₁R₂CR₃R₄-, où R₁ et R₃ sont H, alors R₂ et R₄ peuvent, en outre, former ensemble un groupe -(CH₂)₄-, en partant du trithiocarbonate correspondant, comprenant les étapes suivantes :
a) on traite le trithiocarbonate correspondant de formule (II) 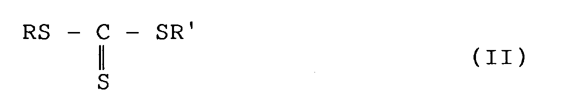 où R et R' sont tels que définis ci-dessus,
avec un composé de formule R''-X, à une température située entre 40°C et 110°C et dans les conditions suivantes :
- quand on souhaite préparer un dithiocarbonate acyclique, R'' = R' = R, et X est Cl, Br, -OSO₂CH₃, -OSO₂CF₃ ou -OSO₂C₆H₄CH₃ ; R''-X est présent en une quantité de 1 à 1,2 mole par mole de trithiocarbonate de formule (II), et on effectue le traitement en présence d'un acide organique, en une quantité de 0,5 à 5 parties en volume pour 1 partie en poids dudit trithiocarbonate, ou en présence d'un mélange d'acide organique/anhydride, en des quantités respectives de 0,5 à 3 et 0,1 à 0,5 parties en volume pour 1 partie en poids dudit trithiocarbonate,
ou vice versa,
- quand on souhaite préparer un dithiocarbonate cyclique, R'' = CH₃, et X est -OSO₂OCH₃ ou -OCOCH₃ ; en outre, quand X est -OSO₂OCH₃, R''-X est présent en une quantité de 1 à 1,2 mole par mole de (II) ; quand X est -OCOCH₃, R''-X est présent en une quantité de 1 à 4 moles par mole de (II), et on effectue ledit traitement en l'absence ou en présence d'un acide organique, en une quantité de 0,5 à 2 parties en volume pour 1 partie en poids de (II), ou d'un mélange d'acide organique/anhydride, et d un acide minéral, en des quantités respectives de 1 à 4, 0,2 à 2 et 0,5 à 4 parties en volume pour 1 partie en poids de (II),
(b) on place le mélange de réaction obtenu dans l'étape a) dans une atmosphère d'azote et on le laisse réagir à une température située entre 40°C et 90°C, après addition d'un mélange d'eau avec un acide minéral, ou d'une solution aqueuse d'un alcool ayant de 1 à 4 atomes de carbone, en une quantité variant entre 2,5 et 20 parties en volume pour 1 partie en poids de trithiocarbonate (II).

2. Procédé selon la revendication 1, caractérisé en ce que, pour obtenir des dithiocarbonates acycliques, dans l'étape a), on traite les trithiocarbonates acycliques avec des composés R''-X choisis parmi les mésylates et les chlorures d'arylméthyle, et l'acide organique utilisé est l'acide trifluoroacétique, seul ou mélangé avec de l'anhydride trifluoroacétique.

3. Procédé selon la revendication 1, caractérisé en ce que, pour obtenir des dithiocarbonates cycliques, dans l'étape a), on mélange le trithiocarbonate cyclique avec R''-X, où X est -OCOCH₃, et l'acide est l'acide sulfurique, l'acide chlorhydrique, l'acide trifluoroacétique ou l'acide trifluorométhanesulfonique.

4. Procédé selon la revendication 1, caractérisé en ce que, quand on souhaite préparer un dithiocarbonate cyclique et qu'on effectue l'étape a) en utilisant du sulfate de diméthyle comme composé R''-X, on effectue la réaction en l'absence de solvants, de diluants ou d'acides.

5. Procédé selon la revendication 1, dans lequel on effectue ledit traitement avec R''-X = sulfate de diméthyle, en présence d'un acide choisi dans le groupe formé par l'acide sulfurique, l'acide chlorhydrique, l'acide trifluoroacétique, l'acide méthanesulfonique et l'acide trifluorométhanesulfonique.

6. Procédé selon la revendication 1, caractérisé en ce que, quand on souhaite préparer un dithiocarbonate cyclique et qu'on effectue l'étape a) en utilisant du sulfate de diméthyle ou de l'acétate de méthyle comme composé R''-X, on effectue la réaction en présence d'un mélange d'acide sulfurique/acide acétique/anhydride acétique, à un rapport en volume variant entre 1:1:1 et 5:5:1.

7. Procédé selon la revendication 1, dans lequel on effectue l'étape b) en utilisant de l'eau mélangée avec de l'acide sulfurique a un rapport volumétrique respectif variant entre 9:1 et 1:1.

8. Procédé selon la revendication 1, dans lequel l'alcool utilisé dans l'étape b) est l'éthanol.

9. Procédé selon la revendication 1, dans lequel, quand un thiol volatil est libéré, il est entraîné par un flux d'azote dans de l'eau saturée de chlore, à une température d'environ 0°C, de manière à être transformé en chlorure d'alcanesulfonyle correspondant.
